(19)

Europäisches
Patentamt
European
Patent Office
Office européen
des brevets

(11) **EP 1 982 646 A2**

(12) # EUROPEAN PATENT APPLICATION

(43) Date of publication:
**22.10.2008 Bulletin 2008/43**

(51) Int Cl.:
**A61B 5/021** (2006.01) **A61B 5/0402** (2006.01)

(21) Application number: **08250089.3**

(22) Date of filing: **09.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **20.04.2007 TW 96114119**

(71) Applicant: **National Research Institute of Chinese Medicine**
**Peitou, Taipei (TW)**

(72) Inventors:
• **Kuo, Terry B.J.**
  **Shilin, Taipei (TW)**

• **Yang, Cheryl C.H.**
  **Shilin, Taipei (TW)**
• **Wu, Hsien-Chang**
  **Songshan, Taipei (TW)**
• **Chen, Hsin-Yo**
  **Songshan, Taipei (TW)**
• **Wu, Tian-Shung**
  **Tainan (TW)**

(74) Representative: **Gee, Steven William**
**D.W. & S.W. GEE**
**1 South Lynn Gardens**
**London Road**
**Shipston on Stour, Warwickshire CV36 4ER (GB)**

(54) **Analysis system and a method for pulse diagnostic in chinese medicine**

(57)    The present application relates to an analysis system and method for pulse diagnosis in Chinese medicine, which analyzes quantitatively the variances of pulse condition, and in particular analyzes the "pulse POSITION", "pulse PACE", "pulse FORM" and "pulse DYNAMICS" elements in pulse condition with remarkable increase in the accuracy of pulse diagnosis. The analysis system for pulse diagnosis of the present application comprises a pulse signal collecting device (10) to collect and generate blood pressure and electrocardiogram signals; and a signal process unit (20) to receive and analyze the blood pressure signal series and electrocardiogram signal series. The execution of the signal process unit includes: establishing the position parameters based on measurement of the blood pressure signals, the heart rate parameters based on calculation of the blood pressure or electrocardiogram signals, the wave form parameters based on analysis of the blood pressure signals, the spectrum analysis parameters based on heart rate variability (HRV) and arterial pressure variability (APV), and the quantitative indexes of the elements in pulse condition of Chinese medicine based on the parameters mentioned above.

FIG. 1

EP 1 982 646 A2

**Description**

BACKGROUND OF THE INVENTION

1. Field of the Invention

The present invention relates to an analysis system and a method for pulse diagnosis in Chinese medicine, in particular relates to an analysis system and a method for pulse diagnosis that is based on quantitative analysis of the elements in pulse conditions.

2. The Prior Arts

Chinese doctors diagnose the whole condition of patients through taking pulse over radial artery at the wrist by finger technique. Pulse diagnosis is used to examine zang-fu organs (viscera), Qi and blood, yin and yang, physiological and pathological condition of human body based on the changes of pulse condition (manifestation). The changes in pulse condition, not only refers to heart beats but also includes 28 different types (qualities) such as stringy (taut, wiry) pulse, slippery pulse, normal pulse, short pulse and so on. The pulse diagnosis is mainly affected by variances of four parameters Wei (pulse POSITION), Shu (pulse PACE), Sing (pulse FORM) and Shi (pulse DYNAMICS), according to ancient Chinese doctor Xu-Hai Zhou. On the basis of "pulse POSITION" the sites of radial artery are divided into three locations: Cun, Guan and Chi. Dr. Zhou proposed that these four parameters should be clearly defined. The pulse POSITION, PACE, FORM and DYNAMICS are the guiding principles for evaluating pulse condition. However, there is no universal standard and simple but scientific ways for pulse diagnosis, and the diagnosis depends on sense of the pulse through sentient fingers, both these made pulse diagnosis difficult to carry out clinically.

Since 1950, the pulse waveforms have been attempted to describe through graphs in the Western world. It is not until the 1980s that the detection device with the advantages of high reproducibility, long term stability, simple pulse-taking and labeling, high sensitivity and wide dynamic range has been developed. During 1970s, the previous arts such as a sphygmometer comprising a pulse sensor, blood pressure transducer, and multi-channel recorder were developed in Taiwan. The pulse wave signal and cardiogram are synchronously displayed when sphygmometer is linked to a computer system. The pulse wave measured using blood pressure transducer was transformed into intensity of resonance wave with different frequencies by Fourier transformation to determine the status of zang-fu organs in another previous art. The drawbacks of the previous arts are costly, human manipulated selection of pulse position, no detailed and objective reports, Chinese doctors-dependent assessment, and not easily understood and need of discussion with Western doctors for related data. According to modem medical studies, the three consisting factors of pulse are the pressure generated by heart beats (cardiac output and contractility), elasticity of artery wall and peripheral resistance, and blood viscosity. Normal pulse is reflected by multi-factors such as heart beat, the rhythm of heart activities, heart eject fraction, artery elasticity, tightness of arterial wall, filling degree of vessels, neural and endocrine regulations.

On the other hand, the physiological information obtained from pulse pressure graph is limited. The artery pulse contains not only the signal of pressure beat, but also capacity of blood vessel, blood flow rate, and vessel 3D movement and so on. The studies of pulse spectrum are mostly restricted within the range of 28 pulse conditions, such as stringy pulse, slippery pulse, racing pulse and so on. Certain pulse spectrum is essentially a certain pulse wave graph of certain composite pulse. Furthermore, there is no significant difference between the pulse pressure graphs of some pulse pressure graph. Therefore, the indexes of pulse pressure graph are focused on "pulse POSITION" and "pulse FORM", which are limited to show quantitatively all the other indexes for pulse condition such as pulse position, pulse width, pulse length, pulse rate, pulse rhythm, pulse force, pulse flow, and pulse hardness/softness. Further studies on pulse condition are needed to operate in coordination.

SUMMARY OF THE INVENTION

The objective of the present invention is to provide an analysis system for pulse diagnosis in Chinese medicine to analyze quantitatively the elements of pulse condition through signal collection, especially the "pulse FORM", "pulse PACE", and "pulse DYNAMICS".

Another object of the present invention is to provide an analysis method for pulse diagnosis in Chinese medicine, which refers to signal series of blood pressure and electrocardiogram to define the quantitative indexes of elements in pulse condition.

Yet another objective of the present invention is to provide an analysis system and a method for pulse diagnosis in Chinese medicine, which defines "pulse FORM", "pulse PACE", and "pulse DYNAMICS" in views of Western medicine.

In order to accomplish the above-mentioned objectives, the present analysis system for pulse diagnosis in Chinese medicine according to the present invention comprises a pulse signal collecting device to collect and generate blood pressure and electrocardiogram signals; and a signal process unit to receive and analyze the blood pressure signal series and electrocardiogram signal series. The execution of the signal process unit includes: establishing the position parameters based on measurement of the blood pressure signals, the heart rate parameters based on calculation of the blood pressure or electrocardiogram signals, the waveform parameters based on analysis of the blood pressure signals, the spectrum analysis parameters based on heart rate variability (HRV) and arterial pressure variability (APV), and the quantitative indexes of the elements in pulse condition of Chinese medicine based on the parameters mentioned above.

It is a further object of the present invention to provide an analysis method for pulse diagnosis in Chinese medicine referring to blood pressure signal series and electrocardiogram signal series. This method comprises: establishing the position parameters based on measurement of the blood pressure signals, establishing the heart rate parameters based on calculation of the blood pressure and electrocardiogram signals, establishing the wave form parameters based on analysis of the blood pressure and electrocardiogram signals, establishing the spectrum analysis parameters based on heart rate variability (HRV) and arterial pressure variability (APV), and the quantitative indexes of the elements in pulse condition of Chinese medicine based on the above-mentioned parameters.

The advantages of the present invention are that it overcomes the quantitative problem of pulse condition through the quantitative indexes with automatic calculation and generates the following merits: provides standard with consistency for the assessment of pulse conditions, prevents human diagnostic errors, increases the accuracy of pulse diagnosis, further enhances effectiveness of the pulse diagnostic device, and combines with Western medicine principles.

The present invention is further explained in the following embodiment, illustration and examples. Those examples below should not, however, be considered to limit the scope of the present invention, it is contemplated that modifications will readily occur to those skilled in the art, which modifications will be within the spirit of the present invention and the scope of the appended claims.

BRIEF DESCRIPTION OF THE DRAWINGS

The related drawings in connection with the detailed description of the present invention are described briefly as follows, in which:

Fig. 1 is a block chart of the analysis system for pulse diagnosis in Chinese medicine of the present invention.

Fig. 2 shows the original blood pressure waveform for 5 seconds according to the present invention. BP, blood pressure; SBP, systolic blood pressure; PPI, pulse-to-pulse interval.

Fig. 3 shows the original blood pressure waveform for 5 min according to the present invention. BP, blood pressure; SBP, systolic blood pressure; PPI, pulse-to-pulse interval.

Fig. 4 is the results of mean systolic blood pressure (MSBP) and mean pulse-to-pulse interval (MPPI) calculated from the blood pressure signals of Fig. 3. HPSD, the power spectrograms of heart rate; BPSD, blood pressure after spectrum analysis; The frequency ranges for the very low-frequency (BVLF, HVLF), low-frequency (BLF, HLF), and high-frequency (BHF, HHF).

DETAILED DESCRIPTION OF THE PREFERRED EMBODIMENT

Definition of the terms in the present invention:

The term "pulse diagnosis" of the present invention indicates that the Chinese physicians examine zang-fu organs (viscera), Qi and blood, yin and yang, physiological and pathological conditions of human body based on the changes of pulse condition (manifestation).

The term "pulse condition" of the present invention indicates that the Chinese physicians feel the variances of a patient's pulse with the finger-tips to judge pulse condition, thus learning and inferring the condition of illness.

The term "pulse POSITION" of the pulse condition according to the present invention indicates the position of the pulse being measured.

The term "pulse PACE" of the pulse condition according to the present invention indicates the number of pulse in unit time.

The term "pulse FORM" of the pulse condition according to the present invention indicates the waveform of the pulse.

The term "pulse DYNAMICS" of the pulse condition according to the present invention indicates the trend of the pulse form, which is the variation in trend.

The term "QRS complex" of the present invention indicates a typical structure on the electrocardiogram showing three separate but tightly closed Q, R, and S waves. Q wave is the initial downward (negative) deflection. R wave is the initial upward deflection following the Q wave, and S wave is a downward deflection following the R wave in the normal electrocardiogram. The QRS complex represents the depolarization of the right and left ventricles.

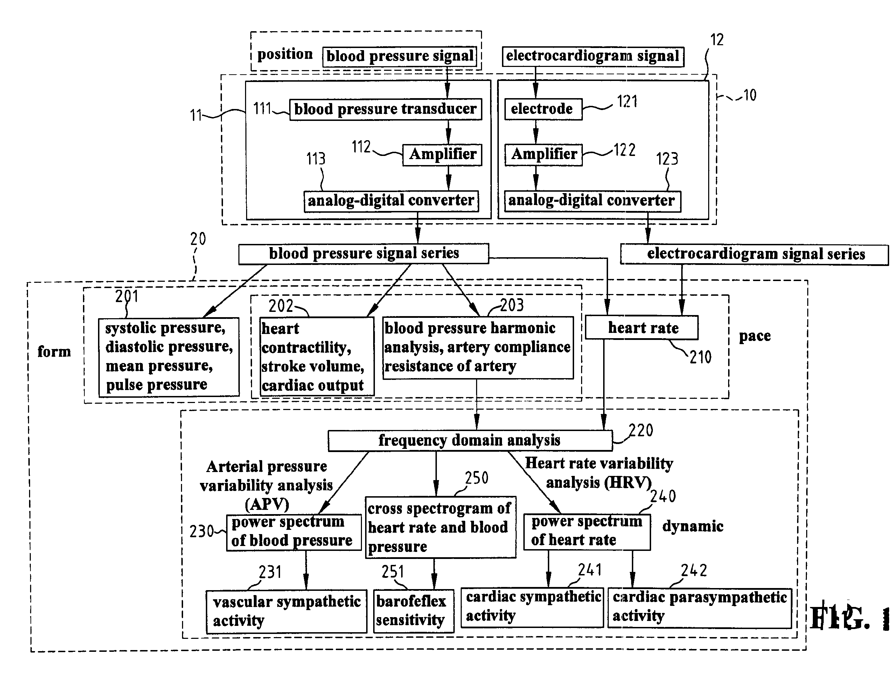

Referring to Fig. 1, a block chart shows the analysis system of the present invention for pulse diagnosis in Chinese medicine. In a preferred embodiment, the analysis system for pulse diagnosis comprises a pulse signal collecting device 10, which further contains a blood pressure signal collecting apparatus 11 and an electrocardiogram signal collecting apparatus 12, while the later is an optional unit in the present invention, wherein the blood pressure signal collecting apparatus 11 collects blood pressure signal from specified regions of human body, such as the positions of Cun, Guan and Chi in radial artery or other regions. The blood pressure signals collected are transformed into electro-signals through blood pressure transducer 111, and amplified through amplifier 112. The amplified signals are converted into digital signals through analog-digital converter 113 at rate of 2048 Hz for several minutes to generate blood pressure signals in digitals. The digital blood pressure signals are measured with time along the horizontal axis to plot the immediate waveform of blood pressure signals.

On the other hand, electrocardiogram signal collecting apparatus 12 collects electrocardiogram signal through electrode 121. The electrocardiogram signals collected are amplified through amplifier 122. The amplified signals are in turn converted into digital signals through analog-digital converter 123 at rate of 256 Hz for several minutes to generate electrocardiogram signals in digitals. The digital electrocardiogram is measured with time along the horizontal axis to plot the immediate waveform of electrocardiogram signals.

A signal process unit 20 is used to receive and analyze the blood pressure signal series and electrocardiogram signal series. The signal process unit 20 can be the micro computer in a personal computer, a PDA (Personal digital assistants), a mobile phone or an electronic watch. The blood pressure signals are collected from specified regions of human body to establish the position parameters and define the position parameters as the "pulse POSITION" element in pulse condition of Chinese medicine.

The systolic pressure is measured at the peak of the waveform of blood pressure signals received from the signal process unit 20, while the diastolic pressure at the nadir in square 201. The value of a mean pressure is obtained through a formula using the systolic and diastolic pressure values (mean pressure= 1/3 systolic pressure + 2/3 diastolic pressure). The value of a pulse pressure is obtained by subtracting diastolic pressure value from the systolic pressure value. In square 202, signal process unit 20 differentiates the digital blood pressure signals (dp/dt) followed by subtracting the minimal value from the maximal value of pulse to obtain the heart contractility. Stroke volume is obtained from the following formula:

$$SV = KP_{sa}(1 + T_s/T_d)$$

wherein K is a constant; $P_{sa}$ is the integrated area of a single pulse pressure curve; and $T_s$ and $T_d$ are the durations of systole and diastole. And cardiac output (SV x HR= CO) is the product of heart rate (HR) and stroke volume (SV). In square 203, the signal process unit 20 processes blood pressure harmonic analysis using fast Fourier transform (FFT) to transform the digital blood pressure signals into spectrum, and estimates the spectral power density by means of integration. Each spectrum is displayed in a two-dimensional histogram or outputs as values to calculate the value of arterial pressure variability (APV). The ultra high frequency component changed with heart beat rate accordingly is the index for harmonics in blood pressure. And artery compliance can be calculated by the following formula:

$$C_p = (SV/K) \times (bexp^{bp}/exp^{bp}_s - exp^{bp}_d).$$

The value of resistance of artery is obtained by the following formula:

$$Z_c = P_f/Q_f$$

wherein $Z_c$ is the resistance of artery, P is the pressure, and Q is the blood flow. The systolic pressure, diastolic pressure, mean pressure and pulse pressure are values from the calculation by signal process unit 20 after receiving the blood pressure waveform signals in square 201. These parameters obtained from analysis of blood pressure waveform are called blood pressure waveform parameters. The signal process unit 20 defines that the blood pressure waveform parameters are the "pulse FORM" element in pulse condition of the Chinese medicine, which uses systolic pressure, diastolic pressure, mean pressure and pulse pressure as quantitative indexes. And heart contractility, stroke volume, cardiac output, blood pressure harmonic analysis, artery compliance and resistance of artery in square 202 and square 203 are quantitative indexes for the "pulse PACE" element and "pulse FORM" element in pulse condition of the Chinese medicine.

On the other hand, the signal process unit 20 receives the blood pressure waveform signals and processes to obtain pulse-to-pulse interval (PPI) value after calculation, which is the heart rate parameter in square 210. Or using electrocardiogram waveform signals to obtain the same heart rate parameter in square 210. The signal process unit 20 defines that the heart rate parameters are the "pulse PACE" element in pulse condition of the Chinese medicine, where heart rate is a quantitative index.

Variability analysis represents the trend or change of any parameter after a long time collection, such as heart rate variability (HRV) obtained after a long time and enough accumulation of heart rate, or arterial pressure variability (APV) obtained after a long time and enough accumulation of arterial pressure.

The heart rate parameters and the blood pressure waveform parameters are analyzed through frequency domain analysis in square 220 in the signal process unit 20. The vascular sympathetic activity in the arterial pressure variability (APV) analysis is obtained from transforming the digital blood pressure signals into spectrum using fast Fourier transform (FFT), and estimating the spectral power density by means of integration to obtain the power spectrum of arterial pressure in square 230. Each spectrum is displayed in a two-dimensional histogram or outputs as values to calculate the value of arterial pressure variability (APV). Low-frequency (BLF) power in square 231 of the arterial pressure variability was quantified to provide an index of vascular sympathetic activity.

The heart rate parameters and the blood pressure waveform parameters are analyzed through frequency domain analysis in square 220 in the signal process unit 20. In the heart rate variability (HRV) analysis, the electrocardiogram signals are transformed into spectrum using fast Fourier transform (FFT), and estimating the spectral power density by means of integration to obtain the power spectrum of heart rate in square 240. Each spectrum is displayed in a two-dimensional histogram or outputs as values to calculate the value of heart rate variability (HRV). The ratio of LF to HF power (LF/HF) in square 241 is quantified to provide an index of cardiac sympathetic activity. HF power is considered to be a pure index of parasympathetic activity while LF power is an index of both sympathetic and vagal modulation in square 242.

The signal process unit 20 defines that spectrum analysis parameters of variability are the "pulse DYNAMICS" element in pulse condition of the Chinese medicine, which uses vascular sympathetic activity, cardiac sympathetic activity, cardiac parasympathetic activity, and baroreflex sensitivity as quantitative indexes.

The values of power spectrum of blood pressure and vascular sympathetic activity in squares 230 and 231 are obtained by means of a continuous time analysis after the signal process unit 20 receives the blood pressure waveform parameters. These parameters using continuous time analysis are the spectrum analysis parameter of APV. The signal process unit 20 defines that spectrum analysis parameter of APV is the "pulse FORM of pulse DYNAMICS" element in pulse condition of the Chinese medicine.

The values of power spectrum of heart rate, cardiac sympathetic activity, and cardiac parasympathetic activity in squares 240, 241 and 242 are obtained by means of a continuous time analysis after the signal process unit 20 receives the heart rate parameters. These parameters using continuous time analysis are the spectrum analysis parameter of heart rate variability (HRV). The signal process unit 20 defines that spectrum analysis parameter of HRV is the "pulse PACE of pulse DYNAMICS" element in pulse condition of the Chinese medicine.

When there is change in blood pressure, heart will adjust the rate accordingly to maintain the consistency of blood pressure since heart rate variability and blood pressure variability are closely linked. Therefore, "pulse FORM of pulse DYNAMICS" and "pulse PACE of pulse DYNAMICS" are responding to each other, and both belong to the system of "pulse DYNAMICS" element in pulse condition. Cross-spectral analysis of heart rate and blood pressure in square 250 and baroreflex sensitivity in square 251 represent the "pulse DYNAMICS" after the integration of "pulse FORM of pulse DYNAMICS" and "pulse PACE of pulse DYNAMICS" analysis. The contents of the signal process unit 20 in spectrum analysis are further explained in the following description.

Arterial Pressure Variability (APV) Analysis

The APV can be collected with a pulse signal collecting device through a blood pressure signal collecting apparatus, such as arm blood pressure monitor, wrist blood pressure monitor, finger plethysmography, pen-style pulse sensors, tonometry, and ring-style blood pressure monitor to collect the waveforms of blood pressure. The signals of blood pressure waveform are amplified 10,000 folds by means of an amplifier and filtered, connected to a signal process unit 20 such as an IBM compatible computer through a 12-bit analog-digital converter (PCL-818L, Advantech, Taiwan), and saved into the signal process unit 20 at the sampling rate of 1024 Hz.

The arterial pressure signal is resampled to maintain time coherence in the signal process unit 20. Firstly, remove the baseline drift to prevent the interference of low frequency. The arterial pressure signal series are divided into blocks (or an analysis window) of 64 seconds (each 4096 data points) to perform fast Fourier transform and estimate the power spectral density. The Hamming window is applied to prevent leakage from frequencies to other spectrum components.

The present invention used differentiation to quantify the power of each frequency component in the power spectral density, including the quantification parameters such as the low frequency power (BLF, 0.04-0.15 Hz) and the high frequency power (BHF, 0.15-0.4 Hz) of the arterial pressure signal spectrum. Low-frequency power of the arterial pressure variability provides an index of vascular sympathetic activity.

Heart Rate Variability (HRV) Analysis

The HRV is the variation of heartbeat intervals as a function of time. Heartbeat intervals can be obtained from collection of the waveform signals of blood pressure by means of a pulse signal collecting device of the present invention, and calculation of these signals after receiving by the signal process unit generate heartbeat interval/heart rate ratio.

On the other hand, heart rate can be collected by recording for at least 5 min through various electrocardiogram signal collecting apparatus 12. The electrocardiogram signals are amplified 1000 folds by means of an amplifier and a filter (0.68-16 Hz), connected to an 8-bit cardiogram analog-digital converter at the sampling rate of 256 Hz.

QRS complex in each heartbeat is recognized as the peak on electrocardiogram signal with a QRS peak detection algorithm. The parameters of amplitude and duration are measured. The mean and standard error are calculated as standard templates.

Each QRS is compared to the standard template. The QRS will be regarded as noise or ectopic beat and deleted when there are three standard errors from the standard template.

The R point will be regarded as the time point for the heartbeat among the qualified QRS complex. The time difference for each heartbeat is regarded as a heartbeat interval (RR interval). Each heartbeat interval is processed with a filter. The calculation of mean and standard error of all heartbeat intervals is performed and confirmed. The error or non-stationary signals will be deleted if the heartbeat intervals are four standard errors from the mean.

The onset time point for R wave is detected with a peak detection algorithm. The heartbeat rate is calculated by the successive reciprocal of the time, and using a sample and hold program to maintain its time coherence. The renewal rate for the sample and hold program is 16 times per second. The continuous heart rate and electrocardiogram signals are divided into blocks (or an analysis window) of 64 seconds (each 1024 data points). An overlap of 50 % in each analysis window is applied to eliminate the low frequency interference by linear drift. The Hamming window is also applied to prevent mutual interferences of spectrum components. And fast Fourier transform is performed to estimate the power spectral density.

The present invention used integration to quantify the power of 2 frequency components in the heart rate spectrum, including low frequency (LF, 0.04-0.15 Hz) and high frequency (HF, 0.15-0.4 Hz). The total power and the ratio of LF to HF (LF/HF) are calculated, wherein HF power is considered to be an index of parasympathetic activity; LF/HF is an index of cardiac sympathetic activity while LF power is an index of both sympathetic and parasympathetic modulation.

APV/HRV Average Periodogram Analysis

The analysis of APV/HRV average periodogram and the APV/HRV transfer function are performed after the data is collected. Arterial pressure and heart rate data are taken for 896 seconds for each analysis. The data can be divided into 55 analysis windows (1024 points per analysis window) with a 50% overlap. The baseline drift in each analysis window is eliminated to prevent the low frequency interference. The Hamming window is also applied to prevent mutual interferences of spectrum components. The spectra of six analysis windows are averaged to obtain average periodogram. The random noises can be decreased after average the data to show components of spectra in good reproducibility.

APV/HRV Cross Spectrogram and Transfer Function Analysis

APV/HRV cross spectrogram analysis is applied after the APV/HRV average periodogram analysis has done. The analysis sources are the same 55 sets of data.

A. Calculates the linear coherence for APV/HRV average periodogram by the following formula:

$$\kappa^2(f) = \Box S_{HB}(f)^2 / [S_{BB}(f) \times S_{HH}(f)]$$

where $S_{BB}(f)$ and $S_{HH}(f)$ represent the power spectrum signals of arterial pressure and heart rate, respectively, and $S_{HB}(f)$ represents their cross spectrogram. The calculation results are distributed in 0 to 1 to show the linear relationship of each frequency between two signals. The reliability of the relative transfer function is also displayed. The statistic significance of the coherence is shown when the value is above 0.5

B. Transfer function:

$$H(f) = S_{HB}(f) / S_{BB}(f)$$

And the amplitude is defined as:

$$\{[H_R(f)]^2 + [H_I(f)]^2\}^{1/2}$$

where the $H_R(f)$ and $H_I(f)$ are the real and imaginary parts of H(f), respectively, and the unit for transfer amplitude is bpm/mmHg.

Assessment of Baroreflex sensitivity

Previous baroreflex studies measure the response of heart rate to rapid changes in blood pressure by disturbing the blood pressure of patients to obtain baroreflex sensitivity. The continuous non-invasive blood pressure and heart rate monitoring of the present invention can analyze the baroreflex sensitivity continuously without disturbing the patients.

Two techniques for evaluating human baroreflex sensitivity have been applied:

A. The AP and HR transfer function method: Baroreflex sensitivity was estimated by the transfer function magnitude between BP and HR. This value can be classified into low-frequency BrrLF and high-frequency BrrHF ranges according to the frequency range measured.

B. The AP and HR sequence technique: Systolic blood pressures are not constant in natural condition. Occurrence of spontaneous fluctuations in blood pressure accompanied with concordant heart rate changes is found. Search for these spontaneous sequences of three or more cycles allows calculating linear regression slope between blood pressure and heart rate changes. Therefore baroreflex sensitivity can be assessed by the slopes of the regression lines of the mean arterial pressure and R-R interval pairs that ascended (BrrA) or descended (BrrD) successively.

The analysis method referring to the blood pressure signal and electrocardiogram signal based on the analysis system of the pulse diagnosis in Chinese medicine in the present invention comprises: establishing the position parameters based on measurement of the blood pressure signals, and defining the position parameters as the "pulse POSITION" element of pulse condition; establishing the heart rate parameters based on calculation of the blood pressure or electrocardiogram signals, and defining the heart rate parameters as the "pulse PACE" element of pulse condition; establishing the blood pressure waveform parameters based on analysis of the blood pressure signals, and defining the blood pressure waveform parameters as the "pulse FORM" element of pulse condition; establishing the spectrum analysis parameters based on heart rate variability (HRV) and arterial pressure variability (APV), and defining the spectrum analysis parameters of variability as the "pulse DYNAMICS" element of pulse condition; and the quantitative indexes of the components in pulse condition of Chinese medicine based on the above-mentioned parameters, where the heart rate is used as a quantitative index for the "pulse PACE" element; mean pressure, systolic pressure, diastolic pressure, and pulse pressure are used as quantitative indexes for the "pulse FORM" element; cardiac output, heart contractility, stroke volume, blood pressure harmonic analysis, artery compliance and resistance of artery are used as quantitative indexes for the "pulse PACE" and "pulse FORM" elements; vascular sympathetic activity, cardiac sympathetic activity, cardiac parasympathetic activity, and baroreflex sensitivity are used as quantitative indexes for the "pulse DYNAMICS" element.

Dialectics of Yin-Yang and Vacuity-Repletion has been used in Chinese medicine to diagnose patients. The constitution and disease of patients are classified into four clinical diagnostics: Yin pattern, Yang pattern, Vacuity pattern and Repletion pattern. The biased Yin-Yang and Vacuity-Repletion of patients can be adjusted to balance and health through the treatment of Chinese medicine. Yin-Yang and Vacuity-Repletion of Chinese medicine are related to each system in human body, which are even indicated in the books of Chinese medicine that they are most related to the so-called autonomic nervous system in Western medicines. However, there is no enough scientific evidence to support such inference.

The analysis method based on the analysis system of the pulse diagnosis in Chinese medicine according to the present invention provides heart rate variability and arterial pressure variability to analyze the information of "pulse DYNAMICS" element by measuring physiological indexes such as mean pressure, systolic pressure, diastolic pressure, pulse pressure, artery compliance, artery resistance and so on. This method removes the quantitative limitation of pulse condition in Chinese medicine, and establishes a preset range, such as 28 pulse conditions, and judging ranges of the above-mentioned parameters corresponded to the elements of pulse condition.

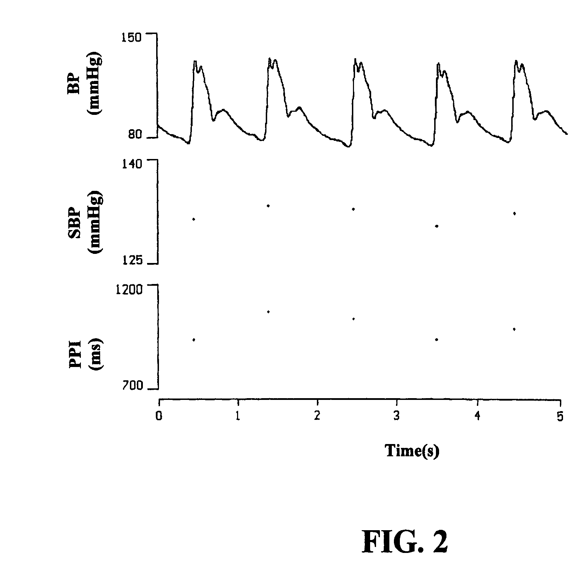

The examples of the calculation results according to the present invention are shown in Fig. 2, Fig. 3, and Fig. 4. Fig. 2 shows the original waveform of blood pressure for 5 seconds. The blood pressure signal of the present invention is shortened to the frequency of 32 Hz through bunching up of eight points to one point. The blood pressure signal series were cut into windows with 32 seconds (1024 points) per window. The blood pressure signals are plotted by points with time scale as the horizontal axis in order to get immediate waveforms of blood pressure. The pulse-to-pulse interval (PPI) and systolic blood pressure (SBP) were derived from this 5 second-waveform of blood pressure (BP). The original blood pressure waveform and the SBP are corresponded to the "pulse FORM" element of pulse diagnosis, while the pulse-to-pulse interval (PPI) to the "pulse PACE" element of pulse diagnosis.

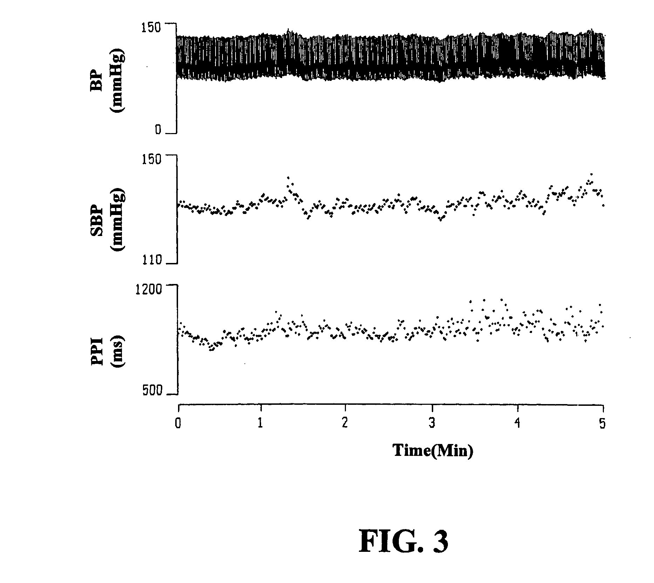

Refer to Fig. 3 for the 5 min original waveform of blood pressure, the pulse-to-pulse interval (PPI) and systolic blood pressure (SBP) were also derived. The original blood pressure waveform and the SBP are corresponded to the "pulse FORM" element of pulse diagnosis, while the pulse-to-pulse interval (PPI) to the "pulse PACE" element of pulse diagnosis. The variability of waveform derived from SBP and from PPI is corresponded to the "pulse DYNAMICS" element of pulse diagnosis.

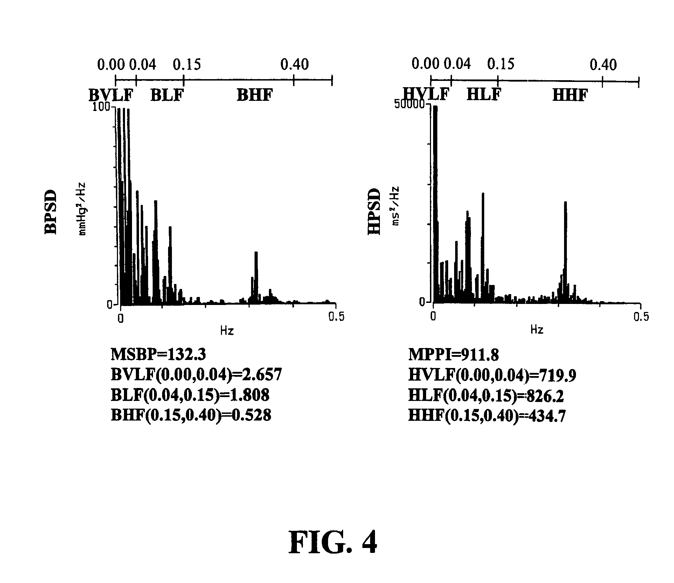

Refer to Fig. 4 for the results of mean SBP (MSBP) and mean PPI (MPPI) derived from the blood pressure signals of Fig. 3, and the power spectrograms of heart rate (HPSD) and blood pressure (BPSD) after spectrum analysis. The top tracing denotes the frequency ranges for the very low-frequency (BVLF, HVLF), low-frequency (BLF, HLF), and high-frequency (BHF, HHF) components of systemic arterial pressure and heart rate signals.

The patients were arranged to be evaluated by experienced doctors of Chinese medicine, and the parameters such as heart rate variability and so on were measured according to the above-mentioned figures. The scientific evidences for dialectic of Yin-Yang and Vacuity-Repletion related to the autonomic nervous system were shown. Firstly, the patients were grouped according to their constitutional types. Regression analysis between various parameters (such as heart rate variability) and constitutional score were performed. Yin-Yang balance was found to correlate positively with sympatho-parasympathetic balance. Patients with Yang pattern had lower parasympathetic activity and higher sympathetic activity than patients with Yin pattern. Patients with Yang-Repletion pattern had higher sympathetic activity than patients with Yang-Vacuity pattern.

The appropriate remedies of Chinese medicine were employed to recuperate the patients' health and constitution. The changes of the subjects were found not only in the dialectic scores, but also the parameters of the autonomic nervous system. The Yin-Yang and vacuity-repletion of Chinese medicine are indeed related to the cardiac autonomic nervous activity. LF/HF can represent the intensity of Yang constitution, the sympathovagal balance represent the Yin-Yang balance, LF represent the intensity of vacuity-repletion constitution, and the recuperation of constitute correlates with the autonomic nervous changes. The values of BVLF, BLF, BHF, HVLF, HLF, and HHF provide the activities of sympathetic and vagus nerves, and also provide the references for Chinese doctors in diagnosis of Yin-Yang and vacuity-repletion patterns.

**Claims**

1.  An analysis system for pulse diagnosis in Chinese medicine comprising:

    a pulse signal collecting device to collect and generate a blood pressure signal; and
    a signal process unit to receive and analyze the blood pressure signal series;

    wherein the execution of the signal process unit including:

    establishing position parameters based on measurement of the blood pressure signals;
    establishing heart rate parameters based on calculation of the blood pressure signals;
    establishing blood pressure waveform parameters based on analysis of the blood pressure signals;
    establishing spectrum analysis parameters based on arterial pressure variability (APV) and heart rate variability (HRV); and
    establishing quantitative indexes of the elements in pulse condition of Chinese medicine based on the parameters mentioned above.

2.  The analysis system for pulse diagnosis in Chinese medicine as claimed in claim 1, wherein the pulse signal collecting device further collects and generates an electrocardiogram signal, and the signal process unit receives and analyzes the electrocardiogram signal series.

3.  The analysis system for pulse diagnosis in Chinese medicine as claimed in claim 2, wherein the execution of the signal process unit further includes establishing heart rate parameters based on calculation of the electrocardiogram signals.

4.  The analysis system for pulse diagnosis in Chinese medicine as claimed in claim 1, wherein the signal process unit defines the position parameters as the "pulse POSITION" element of pulse condition in Chinese medicine.

5.  The analysis system for pulse diagnosis in Chinese medicine as claimed in claim 1, wherein the signal process unit defines the heart rate parameters as the "pulse PACE" element of pulse condition in Chinese medicine.

6.  The analysis system for pulse diagnosis in Chinese medicine as claimed in claim 1, wherein the signal process unit defines the blood pressure waveform parameters as the "pulse FORM" element of pulse condition in Chinese medicine.

7.  The analysis system for pulse diagnosis in Chinese medicine as claimed in claim 1, wherein the signal process unit defines the spectrum analysis parameters of variability as the "pulse DYNAMICS" element of pulse condition in Chinese medicine.

8.  The analysis system for pulse diagnosis in Chinese medicine as claimed in claim 7, wherein the signal process unit defines the spectrum analysis parameters of arterial pressure variability (APV) as the "pulse FORM of pulse DYNAMICS" element of pulse condition in Chinese medicine.

9.  The analysis system for pulse diagnosis in Chinese medicine as claimed in claim 7, wherein the signal process unit defines the spectrum analysis parameters of heart rate variability (HRV) as the "pulse PACE of pulse DYNAMICS" element of pulse condition in Chinese medicine.

10. The analysis system for pulse diagnosis in Chinese medicine as claimed in claim 5, wherein the heart rate, the heart contractility, stroke volume, cardiac output, blood pressure harmonic analysis, artery compliance and resistance of artery are the quantitative index for the "pulse PACE".

11. The analysis system for pulse diagnosis in Chinese medicine as claimed in claim 6, wherein the mean pressure, systolic pressure, diastolic pressure, pulse pressure, heart contractility, stroke volume, cardiac output, blood pressure harmonic analysis, artery compliance and resistance of artery are the quantitative indexes for the "pulse FORM".

12. The analysis system for pulse diagnosis in Chinese medicine as claimed in claim 7, wherein the vascular sympathetic activity, cardiac sympathetic activity, cardiac parasympathetic activity, and baroreflex sensitivity are the quantitative indexes for the "pulse DYNAMICS".

**13.** An analysis method for pulse diagnosis in Chinese medicine referring to blood pressure signal series, wherein comprising:

establishing position parameters based on measurement of the blood pressure signals;
establishing heart rate parameters based on calculation of the blood pressure signals;
establishing blood pressure waveform parameters based on analysis of the blood pressure signals;
establishing spectrum analysis parameters based on arterial pressure variability (APV) and heart rate variability (HRV); and
establishing quantitative indexes of the pulse condition of Chinese medicine based on the parameters mentioned above.

**14.** The analysis method for pulse diagnosis in Chinese medicine as claimed in claim 13, further comprising referring to electrocardiogram signal series.

**15.** The analysis method for pulse diagnosis in Chinese medicine as claimed in claim 14, further comprising establishing heart rate parameters based on calculation of the electrocardiogram signals.

**16.** The analysis method for pulse diagnosis in Chinese medicine as claimed in claim 13, further comprising defining the position parameters as the "pulse POSITION" element of pulse condition in Chinese medicine.

**17.** The analysis method for pulse diagnosis in Chinese medicine as claimed in claim 13, further comprising defining the heart rate parameters as the "pulse PACE" element of pulse condition in Chinese medicine.

**18.** The analysis method for pulse diagnosis in Chinese medicine as claimed in claim 13, further comprising defining the blood pressure waveform parameters as the "pulse FORM" element of pulse condition in Chinese medicine.

**19.** The analysis method for pulse diagnosis in Chinese medicine as claimed in claim 13, further comprising defining the spectrum analysis parameters of variability as the "pulse DYNAMICS" element of pulse condition in Chinese medicine.

**20.** The analysis method for pulse diagnosis in Chinese medicine as claimed in claim 17, further comprising using the heart rate, the heart contractility, stroke volume, cardiac output, blood pressure harmonic analysis, artery compliance and resistance of artery to quantitate the "pulse PACE" element of pulse condition in Chinese medicine.

**21.** The analysis method for pulse diagnosis in Chinese medicine as claimed in claim 18, further comprising using the pulse pressure, mean pressure, systolic pressure, diastolic pressure, the heart contractility, stroke volume, cardiac output, blood pressure harmonic analysis, artery compliance and resistance of artery to quantitate the "pulse FORM" element of pulse condition in Chinese medicine.

**22.** The analysis method for pulse diagnosis in Chinese medicine as claimed in claim 19, further comprising using the vascular sympathetic activity, cardiac sympathetic activity, cardiac parasympathetic activity, and baroreflex sensitivity to quantitate the "pulse DYNAMICS" element of pulse condition in Chinese medicine.

**23.** The analysis method for pulse diagnosis in Chinese medicine as claimed in claim 13, further comprising establishing a preset range of pulse condition and judging the preset range of the pulse condition corresponded to the above-mentioned parameters which in turn corresponded to the elements of pulse condition.

**FIG. 1**

electrocardiogram signal series

electrocardiogram signal
- electrode — 121
- Amplifier — 122
- analog-digital converter — 123
- 12
- 10

blood pressure signal — position
- blood pressure transducer — 111
- Amplifier — 112
- analog-digital converter — 113
- 11

blood pressure signal series — 20

heart rate — 210 — pace

blood pressure harmonic analysis, artery compliance, resistance of artery — 203

heart contractility, stroke volume, cardiac output — 202

systolic pressure, diastolic pressure, mean pressure, pulse pressure — 201 — form

frequency domain analysis — 220

Heart rate variability analysis (HRV) — 240 — dynamic
- power spectrum of heart rate — 241
- cardiac sympathetic activity
- power spectrum of heart rate — 242
- cardiac parasympathetic activity

cross spectrogram of heart rate and blood pressure — 250
- barofeflex sensitivity — 251

Arterial pressure variability analysis (APV) — 230
- power spectrum of blood pressure — 231
- vascular sympathetic activity

10

FIG. 2

EP 1 982 646 A2

FIG. 3

12

EP 1 982 646 A2

0.00 0.04  0.15        0.40

**BVLF  BLF      BHF**

100

BPSD  mmHg²/Hz

0

0                    0.5

Hz

MSBP=132.3
BVLF(0.00,0.04)=2.657
BLF(0.04,0.15)=1.808
BHF(0.15,0.40)=0.528

0.00 0.04  0.15        0.40

**HVLF  HLF      HHF**

50000

HPSD  ms²/Hz

0

0                    0.5

Hz

MPPI=911.8
HVLF(0.00,0.04)=719.9
HLF(0.04,0.15)=826.2
HHF(0.15,0.40)=434.7

# FIG. 4